# EUROPEAN PATENT APPLICATION

(11) **EP 4 660 290 A1**
(43) Date of publication of application: **10.12.2025**
(21) Application number: 24763950.3
(22) Date of filing: 28.02.2024
(51) Int. Cl.: C12M 1/00, C12M 3/00

(54) **CELL TRANSFER METHOD AND CELL TRANSFER DEVICE**

(30) Priority: 28.02.2023 JP 2023029018
(71) Applicant: Terumo Kabushiki Kaisha, Tokyo 151-0072 (JP)
(72) Inventor: LIU, Liming, Ashigarakami-gun, Kanagawa 259-0151 (JP); OKUBO, Itaru, Ashigarakami-gun, Kanagawa 259-0151 (JP)
(74) Representative: TBK
(86) International application number: PCT/JP2024/007197
(87) International publication number: WO 2024/181471

(57) **Abstract**

A cell transfer method includes: a supply step of supplying a culture medium to a bioreactor (24) via a pipe connected to the bioreactor (24) in a state where cells remain in the pipe (first circulation flow path (50)) to transfer the cells remaining in the pipe to the bioreactor (24); an acquisition step of acquiring a predetermined physical quantity related to the culture medium flowing into the bioreactor (24); and a stop step of stopping supply of the culture medium on the basis of a fact that the physical quantity reaches a predetermined threshold value.

## Description

### Technical Field

The present invention relates to a cell transfer method and a cell transfer apparatus for moving cells remaining in a pipe to a bioreactor.

### Background Art

Japanese Patent Application Laid-Open No. 2002-148258 discloses a culture apparatus capable of performing cell culture processing.

### Summary of Invention

For example, there is a cell culture apparatus in which a pipe through which a cell suspension flows is connected to a bioreactor. In order to culture cells using this cell culture apparatus, it is preferable to pack the cells remaining in the pipe into the bioreactor by transferring the cells to the bioreactor before culture. This step is referred to as a cell packing step. In order to optimize the cell culture processing, a technology contributing to optimization of the cell packing step is desired.

An object of the present invention is to solve the above-described problem.

(1) A cell transfer method according to a first aspect of the present invention includes: a supply step of supplying a culture medium to a bioreactor via a pipe connected to the bioreactor in a state where cells remain in the pipe to transfer the cells remaining in the pipe to the bioreactor; an acquisition step of acquiring a predetermined physical quantity related to the culture medium flowing into the bioreactor; and a stop step of stopping supply of the culture medium on the basis of a fact that the physical quantity reaches a predetermined threshold value.
   In the first aspect of the present invention, in the stop step, the supply of the culture medium is stopped on the basis of the fact that the physical quantity is equal to or less than a predetermined threshold value. According to the present invention, the cell packing step can be performed for a minimum necessary time. According to the present invention, the execution time of the cell packing step can be shortened, and the execution time of the cell culture processing can be shortened. Therefore, according to the present invention, the cell culture processing can be optimized. In addition, according to the present invention, the consumption amount of the culture medium can be reduced by shortening the cell packing time.
(2) In the cell transfer method according to the above item (1), the pipe may include a first pipe connected to a first port of the bioreactor and a second pipe connected to a second port of the bioreactor, and in the supply step, the culture medium may be supplied to the bioreactor through the first pipe, and the culture medium may be supplied to the bioreactor through the second pipe.
(3) In the cell transfer method according to the above item (2), in the stop step, the supply of the culture medium may be stopped on the basis of a fact that the physical quantity in the first pipe reaches the threshold value and the physical quantity in the second pipe reaches the threshold value.
(4) In the cell transfer method according to the above items (2) or (3), in the supply step, the culture medium may be supplied to each of the first pipe and the second pipe such that a difference between a timing at which the physical quantity in the first pipe reaches the threshold value and a timing at which the physical quantity in the second pipe reaches the threshold value is equal to or less than a predetermined difference.
   According to the above configuration, the cell packing step can be completed in the shortest time.
(5) In the cell transfer method according to the above item (4), in the acquisition step, the physical quantity may be acquired over time, and in the supply step, the supply of the culture medium to each of the first pipe and the second pipe may be adjusted on the basis of a temporal change in the physical quantity.
(6) In the cell transfer method according to any one of the above items (1) to (5), the physical quantity may be acquired using an optical sensor.
(7) In the cell transfer method according to any one of the above items (1) to (6), the physical quantity may be at least one of an amount of transmitted light transmitted through the culture medium and an amount of scattered light scattered by the culture medium.
(8) A cell transfer apparatus according to a second aspect of the present invention includes: a supply unit that supplies a culture medium to a bioreactor via a pipe connected to the bioreactor in a state where cells remain in the pipe to transfer the cells remaining in the pipe to the bioreactor; an acquisition unit that acquires a predetermined physical quantity related to the culture medium flowing into the bioreactor; and a stop unit that stops supply of the culture medium on the basis of a fact that the physical quantity reaches a predetermined threshold value.
   According to the second aspect of the present invention, an effect equivalent to that of the first aspect of the present invention can be obtained.
(9) In the cell transfer apparatus according to the above item (8), the pipe may include a first pipe connected to a first port of the bioreactor and a second pipe connected to a second port of the bioreactor, and the supply unit may supply the culture medium to the bioreactor through the first pipe, and supply the culture medium to the bioreactor through the second pipe.
(10) In the cell transfer apparatus according to the above item (9), the stop unit may stop the supply of the culture medium on the basis of a fact that the physical quantity in the first pipe reaches the threshold value and the physical quantity in the second pipe reaches the threshold value.
(11) In the cell transfer apparatus according to the above items (9) or (10), the supply unit may supply the culture medium to each of the first pipe and the second pipe such that a difference between a timing at which the physical quantity in the first pipe reaches the threshold value and a timing at which the physical quantity in the second pipe reaches the threshold value is equal to or less than a predetermined difference.
   According to the configuration of the above item (11), an effect equivalent to that of the above item (4) can be obtained.
(12) In the cell transfer apparatus according to the above item (11), the acquisition unit may acquire the physical quantity over time, and the supply unit may adjust the supply of the culture medium to each of the first pipe and the second pipe on the basis of a temporal change in the physical quantity.
(13) In the cell transfer apparatus according to any one of the above items (8) to (12), the physical quantity may be acquired using an optical sensor.
(14) In the cell transfer apparatus according to any one of the above items (8) to (13), the physical quantity may be at least one of an amount of transmitted light transmitted through the culture medium and an amount of scattered light scattered by the culture medium.

According to the present invention, cell culture processing can be optimized.

### Brief Description of Drawings

Fig. 1 is a schematic diagram illustrating a cell culture apparatus.
Fig. 2 is a block diagram of a culture control apparatus (cell transfer apparatus) included in the cell culture apparatus.
Fig. 3 is a flowchart of cell culture processing.
Fig. 4 is a schematic diagram illustrating a cell loading step.
Fig. 5 is a schematic diagram illustrating a cell stirring step.
Fig. 6 is a schematic diagram illustrating a cell packing step.
Fig. 7 is a schematic diagram illustrating a cell culture step.
Fig. 8 is a flowchart of a cell packing step.
Fig. 9 is a time chart illustrating a temporal change in the amount of received transmitted light detected by a sensor unit.

### Description of Embodiments

### [1 Configuration of Cell Culture Apparatus 10]

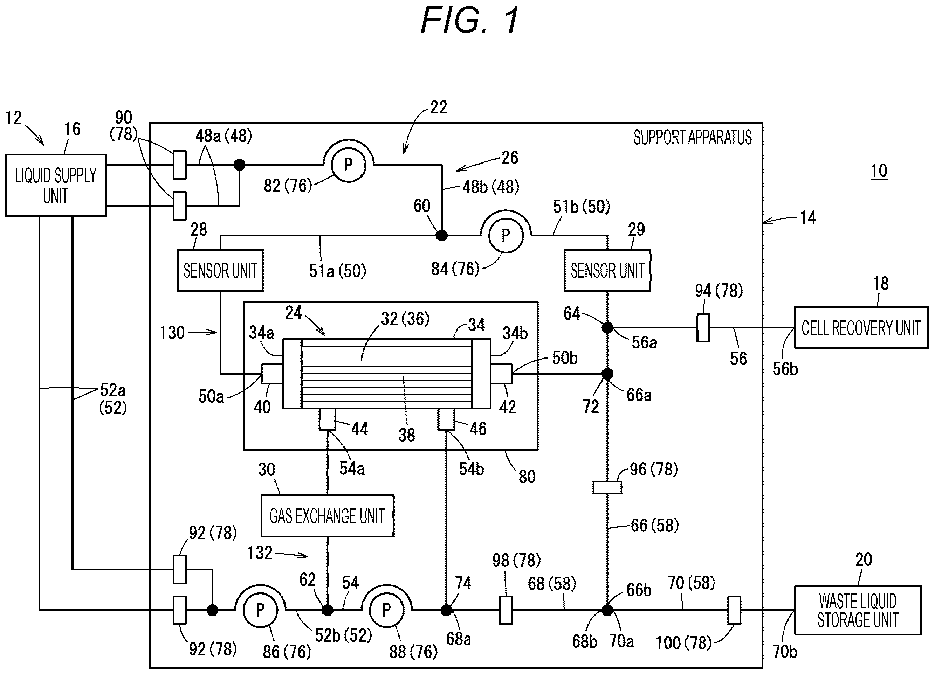

Fig. 1 is a schematic diagram illustrating a cell culture apparatus 10. The cell culture apparatus 10 cultures the cells separated from the biological tissue in a culture medium. The cell cultured in the cell culture apparatus 10 is, for example, an adherent cell or a floating cell. Examples of the cells cultured in the cell culture apparatus 10 include ES cells, iPS cells, and mesenchymal stem cells. Note that the cells cultured by the cell culture apparatus 10 are not limited to the above-described cells.

### [1-1 Cell Culture Circuit 12]

Liquid flows through a cell culture circuit 12. Examples of the liquid include a cell suspension, a culture medium, a washing solution, and a dissociation solution. The cell suspension is a solution containing cells. The culture medium is a culture solution for growing cells.

The culture medium is selected according to the cell to be cultured. As the culture medium, for example, minimum essential media (MEM) are used. The washing solution is a solution for washing the inside of the cell culture circuit 12. Examples of the washing solution include water, a buffer solution, and physiological saline. Examples of the buffer solution include phosphate buffered salts (PBS), tris-buffered saline (TBS), and the like. The dissociation solution is a solution for detaching cells from a bioreactor 24 of the cell culture circuit 12. Examples of the dissociation solution include a trypsin solution and an EDTA solution (ethylenediaminetetraacetic acid solution). Note that the culture medium, the washing solution, and the dissociation solution are not limited to the liquid described above.

The cell culture circuit 12 is a disposable product that is replaced for each cell culture processing. The cell culture circuit 12 includes a liquid supply unit 16, a cell recovery unit 18, a waste liquid storage unit 20, and a culture body 22.

The liquid supply unit 16 includes a plurality of medical bags (not illustrated). Each medical bag is filled with a liquid to be supplied to the culture body 22. For example, the first medical bag is filled with a cell suspension. The second medical bag is filled with a culture medium. The third medical bag is filled with a washing solution. The fourth medical bag is filled with a dissociation solution. Note that each of the plurality of types of culture media may be individually filled in the medical bag.

Each of the cell recovery unit 18 and the waste liquid storage unit 20 includes a medical bag (not illustrated). The cell recovery unit 18 recovers the cells cultured in the culture body 22. The waste liquid storage unit 20 stores the waste liquid generated in the culture body 22.

The culture body 22 includes the bioreactor 24, a flow path 26, sensor units 28 and 29, and a gas exchange unit 30.

The bioreactor 24 comprises a plurality of hollow fiber membranes 32 and a cylindrical housing 34. The plurality of hollow fiber membranes 32 is stored in the housing 34. Each hollow fiber membrane 32 extends along the longitudinal direction of the bioreactor 24. The hollow fiber membrane 32 is made of, for example, a polymer material. The hollow fiber membrane 32 has a plurality of pores (not illustrated). A first end portion of each hollow fiber membrane 32 is fixed to a first end portion 34a in the longitudinal direction of the housing 34. The second end portion of each hollow fiber membrane 32 is fixed to the second end portion 34b in the longitudinal direction of the housing 34.

The bioreactor 24 comprises a first region 36 and a second region 38. The first region 36 is a space inside each hollow fiber membrane 32. The second region 38 is a space between the outer peripheral surface of each hollow fiber membrane 32 and the inner peripheral surface of the housing 34. The first region 36 and the second region 38 communicate with each other through the plurality of pores of each hollow fiber membrane 32.

The housing 34 includes a first port 40, a second port 42, a third port 44, and a fourth port 46. The first port 40 is disposed at the first end portion 34a of the housing 34. The first port 40 is connected to a first end portion of each hollow fiber membrane 32. As a result, the first port 40 communicates with the first region 36. The second port 42 is disposed at the second end portion 34b of the housing 34. The second port 42 is connected to the second end portion of each hollow fiber membrane 32. As a result, the second port 42 communicates with the first region 36.

The third port 44 and the fourth port 46 are disposed on the outer peripheral surface of the housing 34. The third port 44 is disposed between the first port 40 and a central portion of the housing 34 in the longitudinal direction. The fourth port 46 is disposed between the second port 42 and a central portion of the housing 34 in the longitudinal direction. Each of the third port 44 and the fourth port 46 communicates with the second region 38.

The flow path 26 includes a plurality of tubes (pipes) through which liquid flows. Each tube is made of a soft resin material. The flow path 26 includes a first supply flow path 48, a first circulation flow path 50, a second supply flow path 52, a second circulation flow path 54, a collection flow path 56, and a waste liquid flow path 58.

The first supply flow path 48 introduces various liquids of the liquid supply unit 16 into the first circulation flow path 50. The first supply flow path 48 includes a plurality of first upstream flow paths 48a and one first downstream flow path 48b. One first upstream flow path 48a is provided for one medical bag of the liquid supply unit 16. The first upstream flow path 48a is connected to the medical bag of the liquid supply unit 16. In addition, each of the first upstream flow paths 48a is connected to the first downstream flow path 48b. The first downstream flow path 48b is connected to a first merging portion 60 of the first circulation flow path 50.

The first circulation flow path 50 introduces the liquid introduced from the first supply flow path 48 into the bioreactor 24. In addition, the first circulation flow path 50 introduces the liquid discharged from the bioreactor 24 into the bioreactor 24 again. The first end portion 50a of the first circulation flow path 50 is connected to the first port 40 of the bioreactor 24. The second end portion 50b of the first circulation flow path 50 is connected to the second port 42 of the bioreactor 24. The first circulation flow path 50 communicates with an inner hole (first region 36) of each hollow fiber membrane 32. The first merging portion 60 is disposed in the first circulation flow path 50. In the first circulation flow path 50, a portion partitioned by the first end portion 50a and the first merging portion 60 is referred to as a first flow path 51a (first pipe). In the first circulation flow path 50, a portion partitioned by the second end portion 50b and the first merging portion 60 is referred to as a second flow path 51b (second pipe). A collection branch portion 64 is disposed in the second flow path 51b. Furthermore, in the second flow path 51b, a first branch portion 72 is disposed between the collection branch portion 64 and the second end portion 50b.

The second supply flow path 52 introduces various liquids of the liquid supply unit 16 into the second circulation flow path 54. The second supply flow path 52 includes a plurality of second upstream flow paths 52a and one second downstream flow path 52b. One second upstream flow path 52a is provided for one medical bag of the liquid supply unit 16. The second upstream flow path 52a is connected to the medical bag of the liquid supply unit 16. In addition, each of the second upstream flow paths 52a is connected to the second downstream flow path 52b. The second downstream flow path 52b is connected to a second merging portion 62 of the second circulation flow path 54.

The second circulation flow path 54 introduces the liquid introduced from the second supply flow path 52 into the bioreactor 24. In addition, the second circulation flow path 54 introduces the liquid discharged from the bioreactor 24 into the bioreactor 24 again. The first end portion 54a of the second circulation flow path 54 is connected to the third port 44 of the bioreactor 24. A second end portion 54b of the second circulation flow path 54 is connected to the fourth port 46 of the bioreactor 24. The second circulation flow path 54 communicates with a space (second region 38) between the plurality of hollow fiber membranes 32 and the housing 34. The second merging portion 62 is disposed in the second circulation flow path 54. In addition, in the second circulation flow path 54, a second branch portion 74 is disposed between the second merging portion 62 and the second end portion 54b.

The collection flow path 56 introduces the cell suspension discharged from the bioreactor 24 into the cell recovery unit 18. The collection flow path 56 branches from the first circulation flow path 50. A first end portion 56a of the collection flow path 56 is connected to the collection branch portion 64 of the first circulation flow path 50. A second end portion 56b of the collection flow path 56 is connected to the medical bag of the cell recovery unit 18.

The waste liquid flow path 58 introduces the liquid in the first circulation flow path 50 and the second circulation flow path 54 into the waste liquid storage unit 20. The waste liquid flow path 58 includes a first waste liquid flow path 66, a second waste liquid flow path 68, and a third waste liquid flow path 70. The first waste liquid flow path 66 branches from the first circulation flow path 50. A first end portion 66a of the first waste liquid flow path 66 is connected to the first branch portion 72 of the first circulation flow path 50. The second waste liquid flow path 68 branches from the second circulation flow path 54. A first end portion 68a of the second waste liquid flow path 68 is connected to the second branch portion 74 of the second circulation flow path 54. Each of the second end portion 66b of the first waste liquid flow path 66 and the second end portion 68b of the second waste liquid flow path 68 is connected to a first end portion 70a of the third waste liquid flow path 70. A second end portion 70b of the third waste liquid flow path 70 is connected to the medical bag of the waste liquid storage unit 20.

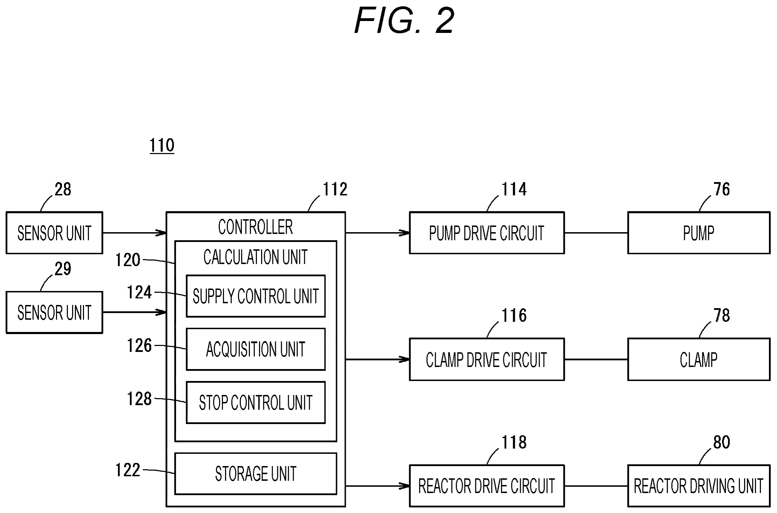

The sensor unit 28 is disposed in the first flow path 51a. On the other hand, the sensor unit 29 is disposed in the second flow path 51b. The sensor unit 28 detects a predetermined physical quantity related to the liquid flowing through the first flow path 51a. The sensor unit 29 detects a predetermined physical quantity related to the liquid flowing through the second flow path 51b. The predetermined physical quantity is a physical quantity proportional to the number of cells in the liquid. For example, each of the sensor units 28 and 29 includes a light source and one or more optical sensors (light receivers). The light source irradiates the liquid with light. The optical sensor receives transmitted light transmitted through the liquid. The optical sensor outputs an electric signal corresponding to the amount of received light to a controller 112 (Fig. 2). Note that the optical sensor may receive scattered light (forward scattered light, side scattered light, backscattered light, and the like) instead of the transmitted light. In addition, each of the sensor units 28 and 29 may include a sensor (for example, a dielectric constant sensor) including two or more electrodes instead of the light source and the optical sensor.

The gas exchange unit 30 is disposed between the second merging portion 62 and the third port 44 in the second circulation flow path 54. The gas exchange unit 30 supplies a gas of a predetermined component to the liquid (culture medium) flowing through the second circulation flow path 54. The gas used in the gas exchange unit 30 has, for example, a component close to air. That is, the gas contains nitrogen, oxygen, and carbon dioxide.

### [1-2 Support Apparatus 14]

The cell culture circuit 12 described above is detachable from the support apparatus 14. The support apparatus 14 includes a cassette that supports the cell culture circuit 12. The support apparatus 14 is a reusable product that can be used a plurality of times.

The support apparatus 14 includes a plurality of pumps 76, a plurality of clamps 78, and a reactor driving unit 80. Each of the plurality of pumps 76, the plurality of clamps 78, and the reactor driving unit 80 includes an electric actuator. Note that each of the plurality of pumps 76, the plurality of clamps 78, and the reactor driving unit 80 may include a fluid actuator.

Each pump 76 can apply a flow force to the liquid in the flow path 26 by squeezing the tube forming the flow path 26. Each pump 76 is operated by power supplied from a pump drive circuit 114 (Fig. 2).

The plurality of pumps 76 includes a first supply pump 82, a first circulation pump 84, a second supply pump 86, and a second circulation pump 88. Each pump 76 functions as follows. Note that, as illustrated in Fig. 1, a state in which the cell culture circuit 12 is attached to the support apparatus 14 is referred to as a "set state".

In the set state, the first downstream flow path 48b is attached to the first supply pump 82. The first supply pump 82 applies a flow force in a direction from the liquid supply unit 16 toward the first circulation flow path 50 to the liquid in the first supply flow path 48.

In the set state, the second flow path 51b of the first circulation flow path 50 is attached to the first circulation pump 84. The first circulation pump 84 applies a flow force in a direction from the second port 42 to the first port 40 to the liquid in the first circulation flow path 50. Note that the first circulation pump 84 can also apply a flow force in a direction from the first port 40 to the second port 42 to the liquid in the first circulation flow path 50.

In the set state, the second downstream flow path 52b is attached to the second supply pump 86. The second supply pump 86 applies a flow force in a direction from the liquid supply unit 16 toward the second circulation flow path 54 to the liquid in the second supply flow path 52.

In the set state, the second circulation flow path 54 is attached to the second circulation pump 88. The second circulation pump 88 applies a flow force in a direction from the fourth port 46 to the third port 44 to the liquid in the second circulation flow path 54. Note that the second circulation pump 88 can also apply a flow force in a direction from the third port 44 to the fourth port 46 to the liquid in the second circulation flow path 54.

Each clamp 78 can close the flow path 26 by compressing the tube forming the flow path 26 in the lateral direction. Each clamp 78 functions as an on-off valve. Each clamp 78 is operated by power supplied from a clamp drive circuit 116 (Fig. 2).

The plurality of clamps 78 includes a plurality of first supply clamps 90, a plurality of second supply clamps 92, a collection clamp 94, a first waste liquid clamp 96, a second waste liquid clamp 98, and a third waste liquid clamp 100. Each clamp 78 functions as follows.

In the set state, one first upstream flow path 48a is attached to one first supply clamp 90. In other words, each of the first upstream flow paths 48a is supported by any one of the first supply clamps 90. The first supply clamp 90 opens and closes the first supply flow path 48.

In the set state, one second upstream flow path 52a is attached to one second supply clamp 92. In other words, each of the second upstream flow paths 52a is supported by any one of the second supply clamps 92. The second supply clamp 92 opens and closes the second supply flow path 52.

In the set state, the collection flow path 56 is attached to the collection clamp 94. The collection clamp 94 opens and closes the collection flow path 56. In the set state, the first waste liquid flow path 66 is attached to the first waste liquid clamp 96. The first waste liquid clamp 96 opens and closes the first waste liquid flow path 66. In the set state, the second waste liquid flow path 68 is attached to the second waste liquid clamp 98. The second waste liquid clamp 98 opens and closes the second waste liquid flow path 68. In the set state, the third waste liquid flow path 70 is attached to the third waste liquid clamp 100. The third waste liquid clamp 100 opens and closes the third waste liquid flow path 70.

The reactor driving unit 80 supports the bioreactor 24. In addition, the reactor driving unit 80 can rotate the bioreactor 24 in one direction and the opposite direction about an axis orthogonal to the longitudinal direction of the bioreactor 24. The reactor driving unit 80 is operated by power supplied from a reactor drive circuit 118 (Fig. 2).

### [1-3 Culture Control Apparatus (Cell Transfer Apparatus) 110]

Fig. 2 is a block diagram of a culture control apparatus 110 included in the cell culture apparatus 10. The culture control apparatus 110 controls each of the plurality of pumps 76, the plurality of clamps 78, and the reactor driving unit 80. Note that the culture control apparatus 110 functions as a cell transfer apparatus that transfers the cells remaining in the first circulation flow path 50 to the bioreactor 24.

The culture control apparatus 110 includes the sensor units 28 and 29, the controller 112, the pump drive circuit 114, the clamp drive circuit 116, and the reactor drive circuit 118. As the controller 112, for example, a computer is used. The controller 112 includes a calculation unit 120 and a storage unit 122.

The calculation unit 120 can be configured by, for example, a processor such as a central processing unit (CPU) or a graphics processing unit (GPU). That is, the calculation unit 120 can be configured by a processing circuit.

The calculation unit 120 includes a supply control unit 124 (supply unit), an acquisition unit 126, and a stop control unit 128 (stop unit). Each of the supply control unit 124, the acquisition unit 126, and the stop control unit 128 can be realized by executing a program stored in the storage unit 122 by the calculation unit 120.

Note that at least a part of the supply control unit 124, the acquisition unit 126, and the stop control unit 128 may be realized by an integrated circuit such as an application specific integrated circuit (ASIC) or a field-programmable gate array (FPGA). Furthermore, at least a part of the supply control unit 124, the acquisition unit 126, and the stop control unit 128 may be configured by an electronic circuit including a discrete device.

The supply control unit 124 performs various controls related to supply of liquid from the liquid supply unit 16 to the bioreactor 24. The acquisition unit 126 acquires the amount of received transmitted light (or the amount of received scattered light) over time on the basis of the electrical signals output from the sensor units 28 and 29. The stop control unit 128 performs various types of control related to stop of supply of liquid from the liquid supply unit 16 to the bioreactor 24.

The storage unit 122 can include a volatile memory (not illustrated) and a non-volatile memory (not illustrated). Examples of the volatile memory include a random access memory (RAM) and the like. The volatile memory is used as a working memory of the processor, and temporarily stores data and the like necessary for processing or operation. Examples of the non-volatile memory include a read only memory (ROM) and a flash memory. The non-volatile memory is used as a memory for storage, and stores a program, a table, a map, and the like. At least a part of the storage unit 122 may be provided in the processor, the integrated circuit, or the like as described above.

The pump drive circuit 114 supplies power to the actuator of the pump 76 to be operated according to a pump operation signal output from the controller 112. Each clamp drive circuit 116 supplies power to the actuator of the clamp 78 to be operated according to the clamp operation signal output from the controller 112. The reactor drive circuit 118 supplies power to the actuator of the reactor driving unit 80 in response to a reactor operation signal output from the controller 112.

### [2 Cell Culture Processing]

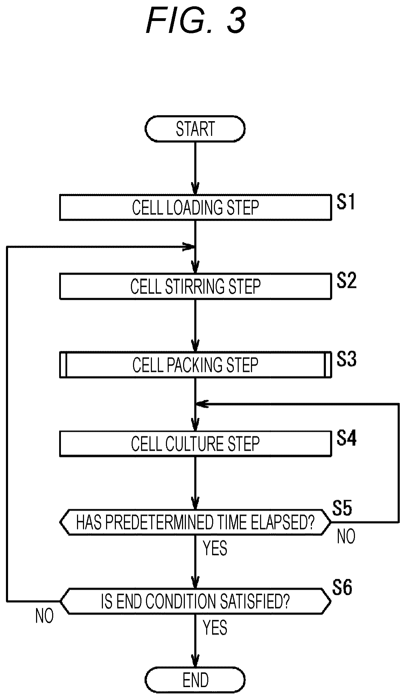

Fig. 3 is a flowchart of cell culture processing. The user uses the input apparatus to perform an operation of starting the cell culture processing. The calculation unit 120 starts the cell culture processing illustrated in Fig. 3 in response to the start operation.

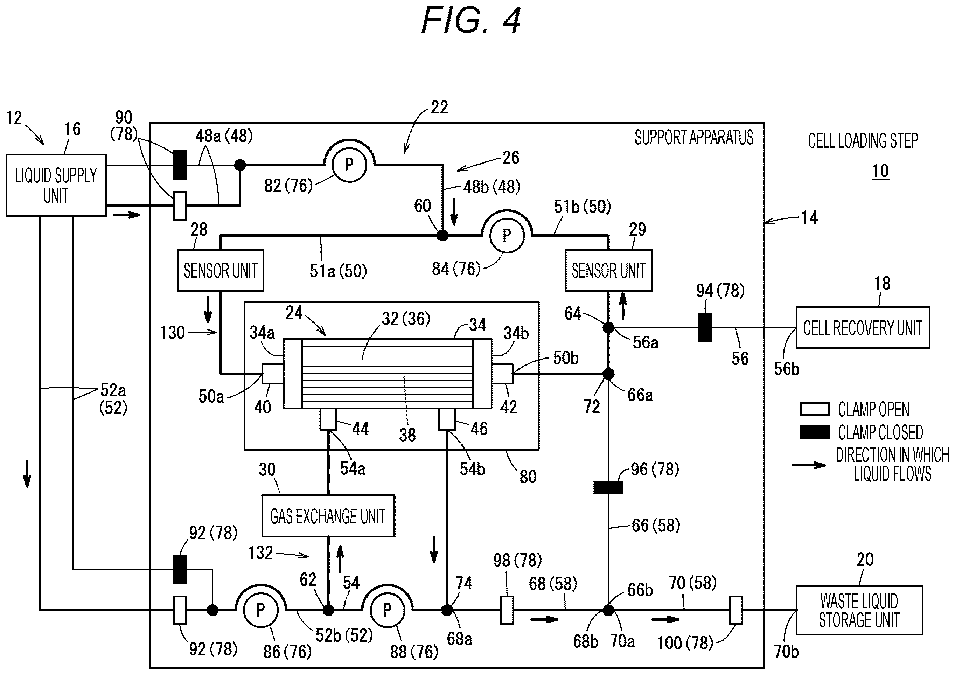

In step S1, the calculation unit 120 performs a cell loading step. The supply control unit 124 outputs a pump operation signal corresponding to the cell loading step to the pump drive circuit 114. In addition, the supply control unit 124 outputs a clamp operation signal corresponding to the cell loading step to the clamp drive circuit 116. Then, the cell culture apparatus 10 enters the state illustrated in Fig. 4. The cell suspension filled in the medical bag of the liquid supply unit 16 flows through the first supply flow path 48 and the first circulation flow path 50 and is supplied to the bioreactor 24. Thus, the cells are introduced into the first region 36 (inside the hollow fiber membrane 32) of the bioreactor 24. The stop control unit 128 stops the cell loading step at a predetermined timing. When step S1 ends, the processing proceeds to step S2.

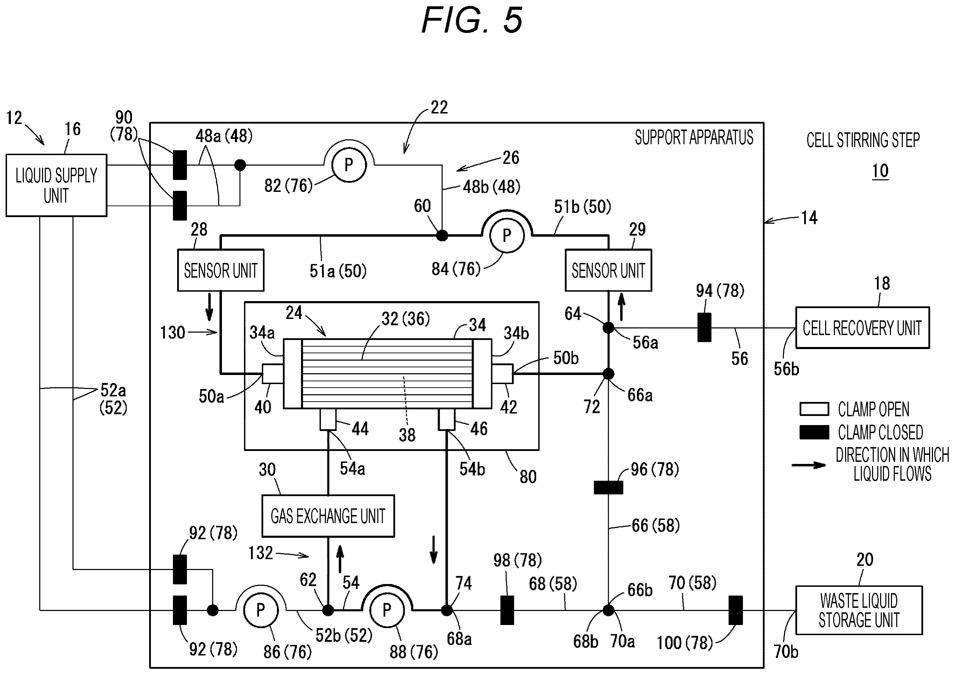

In step S2, the calculation unit 120 performs a cell stirring step. The supply control unit 124 outputs a pump operation signal corresponding to the cell stirring step to the pump drive circuit 114. In addition, the supply control unit 124 outputs a clamp operation signal corresponding to the cell stirring step to the clamp drive circuit 116. Then, the cell culture apparatus 10 enters the state illustrated in Fig. 5. The cell suspension circulates in a circulation path 130 composed of the bioreactor 24 and the first circulation flow path 50. As a result, the cell suspension is stirred, and the cells are diffused within the cell suspension. A state in which cells are uniformly diffused in the cell suspension is referred to as a uniform diffusion state. In a uniform diffusion state, nutrients are evenly distributed among the cells. Note that the supply control unit 124 may output a reactor operation signal for operating the reactor driving unit 80 to the reactor drive circuit 118. The reactor driving unit 80 alternately rotates the bioreactor 24 in one direction and in the opposite direction according to the reactor operation signal. This allows the cell suspension to be homogeneously diffused more quickly. The stop control unit 128 stops the cell stirring step when a predetermined stirring time has elapsed since the start of the cell stirring step. When step S2 ends, the processing proceeds to step S3.

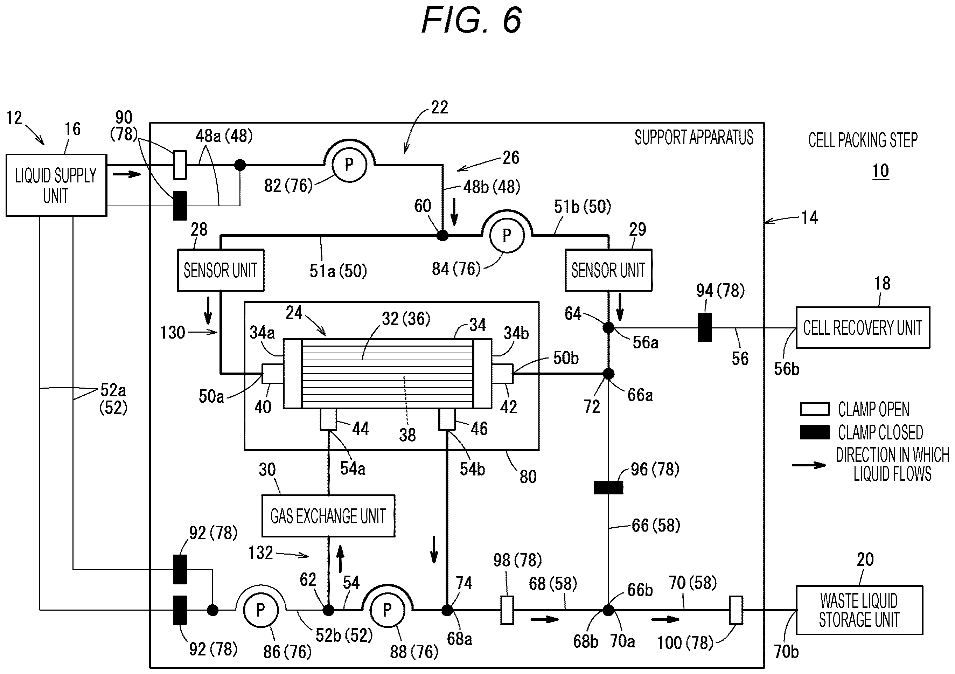

In step S3, the calculation unit 120 performs a cell packing step. The supply control unit 124 outputs a pump operation signal corresponding to the cell packing step to the pump drive circuit 114. In addition, the supply control unit 124 outputs a clamp operation signal corresponding to the cell packing step to the clamp drive circuit 116. Then, the cell culture apparatus 10 enters the state illustrated in Fig. 6. Details of the cell packing step will be described later. The culture medium filled in the medical bag of the liquid supply unit 16 flows through the first supply flow path 48 and the first flow path 51a and is supplied to the bioreactor 24. In addition, the culture medium filled in the medical bag of the liquid supply unit 16 flows through the first supply flow path 48 and the second flow path 51b and is supplied to the bioreactor 24. As a result, the cells remaining in the first circulation flow path 50 move to the first region 36 (inside the hollow fiber membrane 32) of the bioreactor 24. In addition, the culture medium circulates in a circulation path 132 including the bioreactor 24 and the second circulation flow path 54. The excess culture medium is discharged to the waste liquid storage unit 20. When step S3 ends, the processing proceeds to step S4.

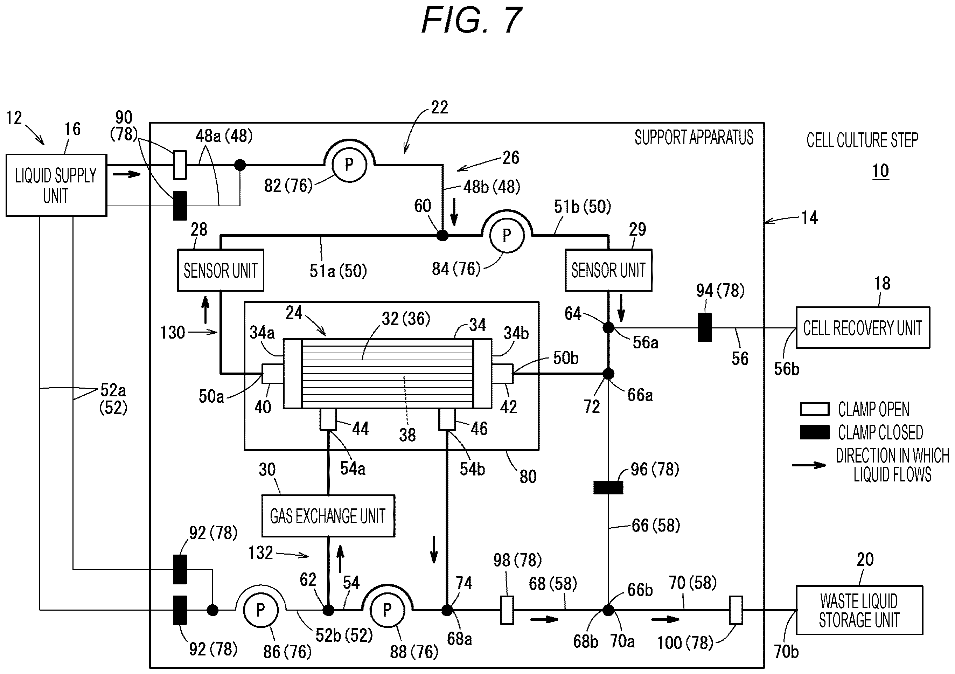

In step S4, the calculation unit 120 performs a cell culture step. Here, the supply control unit 124 outputs a pump operation signal corresponding to the cell culture step to the pump drive circuit 114. In addition, the supply control unit 124 outputs a clamp operation signal corresponding to the cell culture step to the clamp drive circuit 116. Then, the cell culture apparatus 10 enters the state illustrated in Fig. 7. The culture medium filled in the medical bag of the liquid supply unit 16 flows through the first supply flow path 48 and the second flow path 51b, and is supplied to the first region 36 (inside the hollow fiber membrane 32) of the bioreactor 24. In addition, the culture medium circulates in the circulation path 130 including the bioreactor 24 and the first circulation flow path 50. This provides nutrients to the cells. In addition, the culture medium circulates in the circulation path 132 including the bioreactor 24 and the second circulation flow path 54. Note that the excess culture medium is discharged to the waste liquid storage unit 20.

In step S5, the stop control unit 128 determines whether a predetermined culture time (predetermined time) has elapsed since the start of the cell culture step. The storage unit 122 stores a predetermined time in advance. In a case where the predetermined time has elapsed (step S5: YES), the processing proceeds to step S6. On the other hand, in a case where the predetermined time has not elapsed (step S5: NO), the cell culture step of step S4 is continuously performed.

When the processing proceeds from step S5 to step S6, the stop control unit 128 determines whether the end condition of the series of cell culture processing illustrated in Fig. 3 is satisfied. The storage unit 122 stores in advance a calibration curve indicating the relationship between the amount of received transmitted light (or scattered light) of the cell suspension and the number of cells. In addition, the storage unit 122 stores a threshold value as an end condition in advance. The stop control unit 128 estimates the number of cells contained in the cell suspension on the basis of the amount of light received by the sensor unit 28 and the calibration curve. The stop control unit 128 determines that the end condition is satisfied in a case where the estimated number of cells is equal to or greater than the threshold value. Note that the storage unit 122 may store a calibration curve indicating the relationship between the amount of received transmitted light (or scattered light) of the cell suspension and the cell concentration. In a case where the estimated number of cells is equal to or larger than the threshold value (step S6: YES), the series of cell culture processing is ended. On the other hand, in a case where the estimated number of cells is less than the threshold value (step S6: NO), the processing returns to the cell stirring step of step S2.

When the cell culture processing illustrated in Fig. 3 is completed, the supply control unit 124 supplies the dissociation solution to the bioreactor 24, and transfers the cells inside the bioreactor 24 to the cell recovery unit 18.

### [3 Cell Packing Step]

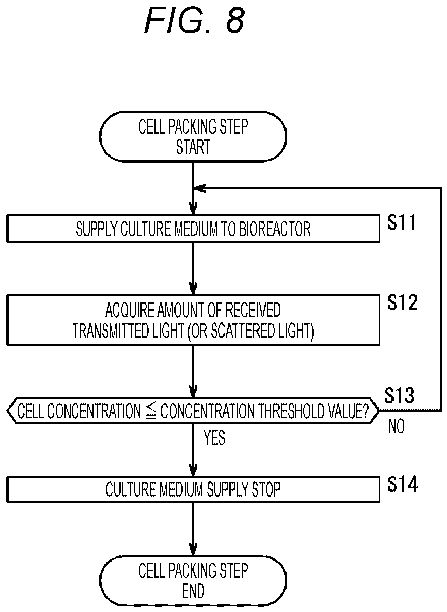

Fig. 8 is a flowchart of a cell packing step. After step S2 illustrated in Fig. 3, a cell packing step described below is performed.

In step S11 (supply step), the supply control unit 124 controls the first supply pump 82 and the first circulation pump 84 to supply the culture medium to the bioreactor 24 via the first supply flow path 48 and the first flow path 51a. In addition, the supply control unit 124 controls the first supply pump 82 and the first circulation pump 84 to supply the culture medium to the bioreactor 24 via the first supply flow path 48 and the second flow path 51b. The cells remaining in the first flow path 51a move to the bioreactor 24 together with the culture medium. The cells remaining in the second flow path 51b move to the bioreactor 24 together with the culture medium. Then, each of the cell concentration in the first flow path 51a and the cell concentration in the second flow path 51b decreases.

Here, the timing at which the cell concentration in the first flow path 51a becomes equal to or less than a predetermined concentration threshold value is referred to as first timing. In addition, the timing at which the cell concentration in the second flow path 51b becomes equal to or less than the predetermined concentration threshold value is referred to as second timing. The supply control unit 124 preferably controls the first supply pump 82 and the first circulation pump 84 such that the difference between the first timing and the second timing is equal to or less than a predetermined difference. Accordingly, the cell packing step can be completed in the shortest time. For example, the ratio between the flow rate of the culture medium supplied from the first supply flow path 48 to the first flow path 51a and the flow rate of the culture medium supplied from the first supply flow path 48 to the second flow path 51b preferably matches the ratio between the volume of the first flow path 51a and the volume of the second flow path 51b. The storage unit 122 stores in advance the target flow rate of the first supply pump 82 and the target flow rate of the first circulation pump 84. The supply control unit 124 controls each of the first supply pump 82 and the first circulation pump 84 so as to achieve the target flow rates stored in the storage unit 122. Note that it is not essential to set the difference between the first timing and the second timing to a predetermined difference or less. That is, the difference between the first timing and the second timing may be larger than the predetermined difference.

In step S12 (acquisition step), the acquisition unit 126 acquires the amount of received transmitted light on the basis of the electric signal output from each of the sensor units 28 and 29. The acquisition unit 126 may acquire the amount of received scattered light instead of the amount of received transmitted light. The acquisition unit 126 acquires a received light amount of each transmitted light every predetermined sampling time.

In step S13, the stop control unit 128 calculates the cell concentration of the culture medium in the first flow path 51a on the basis of the amount of received transmitted light (or scattered light) detected by the sensor unit 28 and the calibration curve. Similarly, the stop control unit 128 calculates the cell concentration of the culture medium in the second flow path 51b on the basis of the amount of received transmitted light (or scattered light) detected by the sensor unit 29 and the calibration curve. Furthermore, the stop control unit 128 compares each calculated cell concentration with a predetermined concentration threshold value. The calibration curve indicates the relationship between the amount of received light and the cell concentration. As the concentration threshold value, an upper limit value of the allowable cell concentration is set. Each of the calibration curve and the concentration threshold value is stored in advance in the storage unit 122. In a case where each cell concentration is equal to or less than the concentration threshold value (step S13: YES), the processing proceeds to step S14. On the other hand, in a case where at least one of the cell concentrations is higher than the concentration threshold value (step S13: NO), the processing returns to step S11. In this case, the processing of step S11 is continued.

When the step proceeds from step S13 to step S14 (stop step), the stop control unit 128 stops the supply of the culture medium to the bioreactor 24. That is, the stop control unit 128 stops the first supply pump 82 and the first circulation pump 84. Upon completion of step S14, the processing proceeds to the cell culture step (step S4) illustrated in Fig. 3.

Note that, in step S13, the stop control unit 128 converts the received light amount into the cell concentration, and performs the stop determination on the basis of the comparison result between the cell concentration and the concentration threshold value. Alternatively, the stop control unit 128 may perform the stop determination on the basis of the comparison result between the amount of received light and the light threshold value. There is a correlation between the amount of received light and the cell concentration. As the amount of received transmitted light increases, the cell concentration decreases. On the other hand, as the amount of received scattered light increases, the cell concentration increases.

### [4 Effects of Present Embodiment]

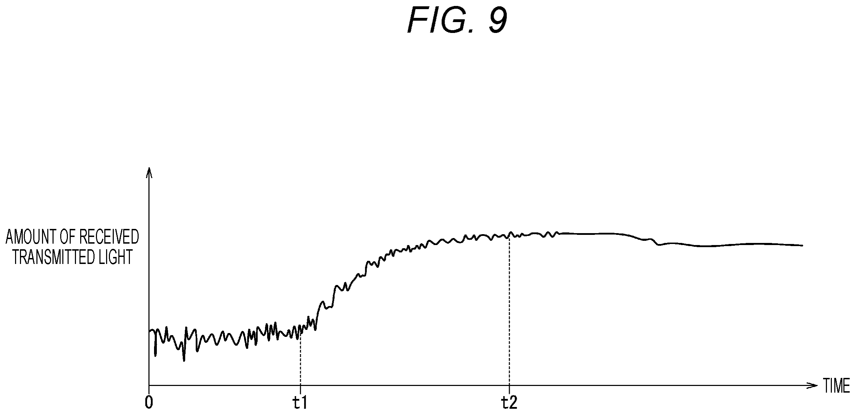

Fig. 9 is a time chart illustrating a temporal change in the amount of received transmitted light detected by the sensor unit 28. Note that the temporal change of the amount of received transmitted light detected by the sensor unit 29 is also similar to that in Fig. 9. As illustrated in Fig. 9, the amount of received transmitted light gradually increases from the time point t1 at which the cell packing step is started. That is, by executing the cell packing step, the number of cells remaining in the first flow path 51a decreases. Then, the turbidity of the culture medium in the first flow path 51a decreases, and the amount of received transmitted light increases. The increase amount per unit time of the amount of received light gradually increases with the lapse of time. The increase amount per unit time of the amount of received light gradually decreases after increasing to some extent, and approaches zero at time point t2. The fact that the increase amount of the amount of received light per unit time approaches zero means that most of the cells in the first flow path 51a have moved to the bioreactor 24.

In the present embodiment, the stop control unit 128 stops the supply of the culture medium on the basis of the fact that the cell concentration (physical quantity) becomes equal to or less than the concentration threshold value (predetermined threshold value). According to the present embodiment, the cell packing step can be performed only for a necessary minimum time. According to the present embodiment, the execution time of the cell packing step can be shortened, and the execution time of the cell culture processing illustrated in Fig. 3 can be shortened. Therefore, according to the present embodiment, the cell culture processing can be optimized. In addition, according to the present embodiment, the consumption amount of the culture medium can be reduced by shortening the cell packing time.

### [5 Other Embodiments]

Only one of the sensor units 28 and 29 may be provided in the cell culture circuit 12. In this case, in step S11, the supply control unit 124 controls the first supply pump 82 and the first circulation pump 84 so that the first timing at which the cell concentration in the first flow path 51a becomes equal to or less than the predetermined concentration threshold value and the second timing at which the cell concentration in the second flow path 51b becomes equal to or less than the predetermined concentration threshold value are substantially the same.

Note that, the present invention is not limited to the above disclosure, and various configurations can be adopted without departing from the gist of the present invention.

## Claims

1. A cell transfer method comprising:
a supply step of supplying a culture medium to a bioreactor via a pipe connected to the bioreactor in a state where cells remain in the pipe to transfer the cells remaining in the pipe to the bioreactor;
an acquisition step of acquiring a predetermined physical quantity related to the culture medium flowing into the bioreactor; and
a stop step of stopping supply of the culture medium on a basis of a fact that the physical quantity reaches a predetermined threshold value.

2. The cell transfer method according to claim 1, wherein
the pipe includes a first pipe connected to a first port of the bioreactor and a second pipe connected to a second port of the bioreactor, and
in the supply step, the culture medium is supplied to the bioreactor through the first pipe, and the culture medium is supplied to the bioreactor through the second pipe.

3. The cell transfer method according to claim 2, wherein
in the stop step, the supply of the culture medium is stopped on a basis of a fact that the physical quantity in the first pipe reaches the threshold value and the physical quantity in the second pipe reaches the threshold value.

4. The cell transfer method according to claim 2, wherein
in the supply step, the culture medium is supplied to each of the first pipe and the second pipe such that a difference between a timing at which the physical quantity in the first pipe reaches the threshold value and a timing at which the physical quantity in the second pipe reaches the threshold value is equal to or less than a predetermined difference.

5. The cell transfer method according to claim 4, wherein
in the acquisition step, the physical quantity is acquired over time, and
in the supply step, the supply of the culture medium to each of the first pipe and the second pipe is adjusted on a basis of a temporal change in the physical quantity.

6. The cell transfer method according to any one of claims 1 to 5, wherein
the physical quantity is acquired using an optical sensor.

7. The cell transfer method according to claim 6, wherein
the physical quantity is at least one of an amount of transmitted light transmitted through the culture medium and an amount of scattered light scattered by the culture medium.

8. A cell transfer apparatus comprising:
a supply unit that supplies a culture medium to a bioreactor via a pipe connected to the bioreactor in a state where cells remain in the pipe to transfer the cells remaining in the pipe to the bioreactor;
an acquisition unit that acquires a predetermined physical quantity related to the culture medium flowing into the bioreactor; and
a stop unit that stops supply of the culture medium on a basis of a fact that the physical quantity reaches a predetermined threshold value.

9. The cell transfer apparatus according to claim 8, wherein
the pipe includes a first pipe connected to a first port of the bioreactor and a second pipe connected to a second port of the bioreactor, and
the supply unit supplies the culture medium to the bioreactor through the first pipe, and supplies the culture medium to the bioreactor through the second pipe.

10. The cell transfer apparatus according to claim 9, wherein
the stop unit stops the supply of the culture medium on a basis of a fact that the physical quantity in the first pipe reaches the threshold value and the physical quantity in the second pipe reaches the threshold value.

11. The cell transfer apparatus according to claim 9, wherein
the supply unit supplies the culture medium to each of the first pipe and the second pipe such that a difference between a timing at which the physical quantity in the first pipe reaches the threshold value and a timing at which the physical quantity in the second pipe reaches the threshold value is equal to or less than a predetermined difference.

12. The cell transfer apparatus according to claim 11, wherein
the acquisition unit acquires the physical quantity over time, and
the supply unit adjusts the supply of the culture medium to each of the first pipe and the second pipe on a basis of a temporal change in the physical quantity.

13. The cell transfer apparatus according to any one of claims 8 to 12, wherein
the physical quantity is acquired using an optical sensor.

14. The cell transfer apparatus according to claim 13, wherein
the physical quantity is at least one of an amount of transmitted light transmitted through the culture medium and an amount of scattered light scattered by the culture medium.
